# EUROPEAN PATENT APPLICATION

(11) **EP 4 417 247 A1**
(43) Date of publication of application: **21.08.2024**
(21) Application number: 23156916.1
(22) Date of filing: 15.02.2023
(51) Int. Cl.: A61N 1/36, A61B 5/12, A61N 1/40, A61N 2/00

(54) **SYSTEM AND METHOD FOR TREATMENT OF TINNITUS BY BIORESONANCE**

(71) Applicant: VV Analytics, 5030 Gembloux (BE)
(72) Inventor: HAVELANGE, Gautier, 5030 Gembloux (BE)
(74) Representative: Calysta NV

(57) **Abstract**

System for treating a patient having tinnitus, comprising: an EM generator (1) configured to generate an EM stimulation for the patient, wherein the EM stimulation is an electric and/or magnetic stimulation, wherein the EM generator (1) is configured to control an EM frequency of the EM stimulation; and a control means (2) configured to receive a tinnitus frequency (F) of the patient, to determine the EM frequency based on the received tinnitus frequency (F), and to control the EM generator (1) to generate an EM stimulation based on the computed EM frequency.

## Description

### Technical Field

The present invention relates to a system and method for treatment of tinnitus.

### Prior art

Tinnitus is the perception of sound when no corresponding external sound is present. 95% of tinnitus cases are not related to a physical cause of the ear, eardrum and auditory nerve. They are called "subjective tinnitus".

There are currently many treatments proposed against tinnitus.

WO2022/201058 discloses a treatment including among others an electromagnetic (EM) stimulation using inductive means inducing in the area of the acoustic nerve a micro-electric stimulation which causes a light hyperemia and interferes with the transmission of the disorder to the brain. Also, EP3537972 and CN104490517 discloses as well to stimulate the ear with low or medium frequency EM waves. Others disclose a stimulation with pulsed EM waves like in CN212730731, EP2456350, EP3393578. KR101342083B1 and EP3416719 discloses the stimulation by an electrical signal applied via electrodes.

Some treatments identify the sound frequency (tone) of tinnitus (tinnitus frequency) of the patient, in order to create an acoustic wave based on the identified tinnitus frequency. This can be a white noise or piece of music from which the tinnitus frequency is removed. The white noise or music without the tinnitus frequency is replayed for the patient to treat the tinnitus.

Unfortunately, the existing treatment methods do not provide a satisfying result.

### Brief summary of the invention

It is the object of the invention to propose a new treatment method and system against tinnitus.

According to the invention, this object is solved by the system and method according to the independent claims.

According to the invention, this object is solved by the system for treating a patient having tinnitus, comprising an EM generator and a control means, wherein the EM generator is configured to generate an EM stimulation for the patient, wherein the EM stimulation is an electric and/or magnetic stimulation, wherein the EM generator is configured to control an EM frequency of the EM stimulation, wherein the control means is configured to receive a tinnitus frequency of the patient, to determine the EM frequency based on the received tinnitus frequency, and to control the EM generator to generate an EM stimulation based on the computed EM frequency.

According to the invention, this object is solved by the method for treating a patient having tinnitus, comprising the following steps: measure a tinnitus frequency of the patient; determine the EM frequency based on the measured tinnitus frequency; and generate an EM stimulation based on the computed EM frequency, wherein the EM stimulation is an electric and/or magnetic stimulation.

By determining the EM frequency of the EM stimulation of the tinnitus suffering ear based on the tinnitus frequency, i.e. based on the frequency of the tone the patient hears, the treatment by EM stimulation is further improved compared to known EM stimulation which only apply standard EM frequencies without considering the actual frequency of the tinnitus frequency. As the tinnitus is very often related to hearing stress, the training or re-information of the brain with the tinnitus frequency which normally creates the stress proofed to reduce the tinnitus. It showed that the direct EM stimulation of the brain based on EM frequencies based on the tinnitus frequency has a much better effect than training the brain with a corresponding audio signal based on the tinnitus signal. A further advantage of the stimulation with EM stimulations instead of audio stimulations, the EM stimulations can modulate audible frequencies with frequencies outside of the audible range.

The dependent claims refer to further advantageous embodiments.

In one embodiment, the EM frequency determined comprises the same frequency value in an EM frequency spectrum of the EM stimulation than the frequency value of the tinnitus frequency in a sound frequency spectrum, wherein the same frequency value in the EM frequency spectrum is called EM tinnitus frequency. The use of the corresponding frequency of the tinnitus tone as EM frequency of the EM stimulation showed the best treatment result for tinnitus. Contrary to the state of the art, the brain is stimulated exactly with the frequencies the patient hears. This allows to get the brain used to this kind of stress and reduce the effect of the tinnitus.

In one embodiment, the EM frequency determined comprises a frequency range around the EM tinnitus frequency. This has the advantage to compensate for measurement errors of the tinnitus frequency F due to limits of the sensitivity of the ear. The frequency range of non-zero EM frequencies around the EM tinnitus frequency assures that the frequency range contains the exact frequency of the tinnitus.

In one embodiment, the frequency range around the EM tinnitus frequency range is larger, if the EM tinnitus frequency is above a certain frequency, compared to a frequency range, when the EM tinnitus frequency is below this certain frequency. This can be realized by a function which increases (continuously) the frequency range for increasing EM tinnitus frequencies. This can also be realized by a step function which increases the frequency range above a certain threshold frequency. This embodiment assures that the frequency range increases for increasing frequencies where the measurement error becomes higher as the sensitivity of the ear decreases.

In one embodiment, the EM frequency determined comprises the EM tinnitus frequency and at least one harmonic frequency of the EM tinnitus frequency. For some patients the inclusion of the harmonics of the EM tinnitus frequency (in addition to the EM tinnitus frequency) further improved the treatment result.

In one embodiment, the EM generator comprises an antenna for generating an EM wave.

In one embodiment, the EM generator comprises at least one electrode to be brought in contact with the patient for applying an electric signal to the head of the patient.

In one embodiment, the EM generator comprises a capacitive coupling means for inducing an electric field in the head of the patient and/or an inductive coupling means for inducing a magnetic field in the head of the patient.

In one embodiment, the EM generator is integrated in noise cancelling headphones. This has the advantage that the ear and the auditory cortex is not disturbed during the treatment by external sound. So, the auditory system can focus fully on the treatment.

In one embodiment, the control means is realized by a computing device with an audio output, wherein the computing device contains a software program configured, when executed on a processing means of the computing means, for outputting an audio signal with the determined EM frequency at the audio output, wherein the EM generator is connected to the audio output to generate the EM stimulation based on the audio signal output from the audio output. This allows to use any standard computing device to realize the system by a dedicated software and using an EM generator connected to the audio output of the computing device like a peripheral device. The dedicated software can be based mainly on audio software components and are very easy to program using existing software modules.

In one embodiment, the audio output is connected via a cable to the EM generator, wherein the audio output is configured to output the audio signal as analogue audio signal, wherein EM generator is driven by a signal based on the analogue audio signal.

In one embodiment, the computing device comprises a smartphone with the audio output.

In one embodiment, the computing device is configured to generate an audio file based on the determined EM frequency, wherein the computing device is configured to replay the audio file to give out the audio signal to the audio output.

In one embodiment, the computing device comprises a client server system with a server and at least one client device, wherein the at least one client device contains the audio output connected to the EM generator. Preferably, the client device is a smartphone or tablet running a special software for the treatment of the tinnitus including the connection to the server and the control of the EM generator. Preferably, the server comprises a user management for the management of different users authenticated by user credentials, wherein the client device is associated with one of the users based on the user credentials received at the client device. Preferably, the tinnitus frequency and/or the EM frequency determined is stored at the server in relation to the user.

In one embodiment, the system comprising a tinnitus measurement means configured to measure the tinnitus frequency of the patient for providing the control means with the tinnitus frequency.

In one embodiment, the tinnitus measurement means is realized as a measurement software in the computing device.

In one embodiment, the measurement means/software is configured to output different audio tones in the audible frequency range and to receive a user input confirming that a certain tone corresponds to the tinnitus frequency.

In one embodiment, the EM frequency comprises the EM tinnitus frequency modulated with a frequency between 0,5 and 100Hz.

In one embodiment, the EM tinnitus frequency is modulated with a modulation frequency between 5Hz and 15Hz, preferably between 7 Hz and 13 Hz, preferably between 8 Hz and 12 Hz. Such a modulation frequency corresponds to the frequency of the alpha waves which is the wave frequency present in the brain when the people is relaxed. As the tinnitus frequency is associated with a stress, the modulation of the EM frequency with the alpha waves allows to reinform the brain that the EM frequency related to the tinnitus frequency is actually not related to stress but to relax. This modulation frequency showed in tests the best results among all.

In one embodiment, the EM tinnitus frequency is modulated with a modulation frequency between 0.5Hz and 2Hz, preferably between 0.8 Hz and 1.5 Hz, preferably between 0.9 Hz and 1.2 Hz. Such a modulation frequency corresponds to the frequency of the heart rate. Also, this modulation showed a further improvement of the treatment.

In one embodiment, the EM stimulation is performed during one or more other therapeutic acts. Other embodiments according to the present invention are mentioned in the appended claims and the subsequent description of an embodiment of the invention.

### Brief description of the Drawings

Fig. 1 shows a system for treatment against tinnitus according to the invention.
Fig. 2 shows a schematic embodiment of the EM generator 1 of Fig. 1.
Fig. 3 shows a second embodiment of the EM generator 1 of Fig. 1.
Fig. 4 shows a third embodiment of the EM generator 1 of Fig. 1.
Fig. 5 shows a fourth embodiment of the EM generator 1 of Fig. 1.
Fig. 6 shows a first realization of the system according to the invention.
Fig. 7 shows a second realization of the system according to the invention.
Fig. 8 shows a first embodiment of the tinnitus measurement means 3 of Fig. 1.
Fig. 9 shows the steps of the method according to the invention.

In the drawings, the same reference numbers have been allocated to the same or analogue element.

### Detailed description of an embodiment of the invention

Other characteristics and advantages of the present invention will be derived from the non-limitative following description, and by making reference to the drawings and the examples.

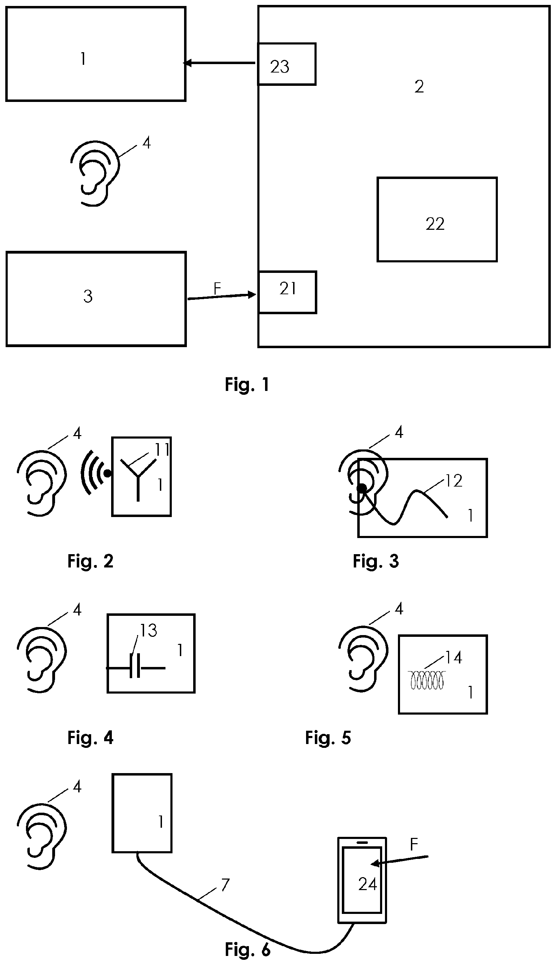

Fig. 1 shows a system for treatment of tinnitus of a patient. The system comprises an EM generator 1 and a control means 2. Optionally, the system can comprise a tinnitus measurement means 3.

The EM generator 1 is configured to generate an EM stimulation the patient suffering the tinnitus. The EM stimulation is applied to the ear 4 of the patient or more generally to the head of the patient so that the brain creating the tinnitus in the ear 4 of the patient is treated by the EM stimulation.

The EM stimulation is an electric and/or magnetic stimulation. The EM stimulation is an electric and/or magnetic stimulation directly generated by the EM generator 1, i.e. not biologically generated by the ear 4 converting a sound wave received at the ear 4. Thus, the EM stimulation is not obtained by converting a sound/pressure wave into an electric signal in the ear, but by generating the EM stimulation, e.g. the electric signal applied or the electric field directly by the EM generator 1. As shown in Fig. 2 to 4, the EM stimulation can be realized by an electromagnetic wave (Fig. 2), an electric signal applied via an electrode 12 (Fig. 3), a capacitive coupling (Fig. 4) or a magnetic coupling (Fig. 5). The electromagnetic wave is generated preferably by an antenna 11 as shown in Fig. 2. The antenna 11 is hold close to the ear 4 when generating the EM stimulations so that the electromagnetic wave emitted radiates in the ear 4 and generates the EM stimulation in the ear 4 by the interaction of the electromagnetic wave with the nerves of the ear 4. Such an EM stimulation could also be achieved by applying an electric signal directly via an electrode 12 to the ear 4 of the patient. The electrodes are preferably applied on the skin of the patient in the vicinity of the ear 4. However, they could also be applied by an invasive electrode. The EM stimulation could also be achieved by a capacitive coupling via a capacitor 13 as shown in Fig. 4. The capacitor 13 generates an electric field which generates the EM stimulation in the ear 3. The EM stimulation could also be achieved by a magnetic coupling via an inductor 14 as shown in Fig. 5. The inductor 14 generates a magnetic field which generates the EM stimulation in the ear 3. Similar as the antenna 11, the capacitor 13 or the inductor 14 are hold close to the ear 4 so that the electric or magnetic field penetrates the ear 4 and generates the treatment effect in the ear 4. Thus, the EM generator 1 comprises an EM conversion means like 11, 12, 13 or 14 to convert an electric signal into an EM stimulation in the ear 4. Instead of applying the EM stimulation to the ear 4, it is also possible to apply the EM stimulation to other parts of the head of the patient, for example close the auditory cortex.

The EM stimulation is realized with an EM frequency. The EM frequency is a frequency in the audio frequency range, i.e. between 20Hz and 20 kHz. The EM frequency can comprise also a plurality of frequencies, e.g. a base frequency and one or more of its harmonics and/or a combination of different tones. The EM frequency as will be explained later in more detail will be selected based on an input, namely based on the frequency value of the tinnitus frequency received, i.e. of the tinnitus tone heard by the patient.

The magnitude of the EM stimulation must not be big to show an effect. First experiments with electromagnetic waves lower than 0,1 V/m did show very promising results. Such electric field strengths are much lower what is normally experienced by the use of electronic devices in our daily live.

The EM stimulation is realized preferably by applying an analogue electric signal to the antenna 11, the electrode 12, the capacitor 13 or the magnetic inductor 14 of the EM generator 1. Since only very low electric currents are necessary and since the EM frequencies in the audio frequency range are desired, preferably the electric signal driving the EM generator 1 is preferably an audio signal. An audio signal is an electric signal configured to be replayed by a headphone or other type of speaker. However, instead of applying the audio signal on a speaker, it is applied to the EM conversion means to convert the audio signal into an EM stimulation of in the ear 4. Preferably, the audio signal is generated with a standard audio output equipment, like an audio jack connector and its driving circuit.

The control means 2 is configured to receive a tinnitus frequency of the patient, to determine the EM frequency based on the received tinnitus frequency, and to control the EM generator to generate an EM stimulation based on the computed EM frequency.

The control means 2 comprises preferably an input means 21 for receiving the tinnitus frequency F. The input means 21 can be a user input means for receiving the tinnitus frequency F as a user input. The user input means can be a touch screen, a mouse, a keyboard or any other user input means. It is also possible that the input means 21 is a communication interface receiving the tinnitus frequency from the tinnitus measurement means 3. The tinnitus frequency is the audio frequency at which the patient hears the perceived sound of the tinnitus in the ear 4 which should be treated. In other words, the tinnitus frequency corresponds to the tone the patient perceives in its ear 4. The tinnitus frequency can comprise a plurality of different frequencies, i.e. a plurality of tones. For example, the tinnitus frequency can comprise a base frequency/tone and one or more of its harmonics. For example, the tinnitus frequency can comprise a complex sound consisting of a plurality of different tones.

The control means 2 comprises a processing means 22 for determining the EM frequency based on the received tinnitus frequency. The EM frequency is preferably determined as comprising the same frequency value as the tinnitus frequency. For example, if the tinnitus frequency received comprises one frequency, i.e. one tone, the EM frequency is determined as having the same frequency. If the tinnitus frequency received comprises a plurality of frequencies or tones, the EM frequency is determined as having the same plurality of frequencies as the tinnitus frequencies received. Even though the best results were obtained by EM frequencies including the same frequency value of the tinnitus frequency F, good results were also achieved when the EM frequency contains (only) harmonics of the tinnitus frequency F. In general, it seems that the treatment with EM stimulation at an EM frequency determined as a function of the tinnitus frequency F show a particularly good result. Thus, according to the invention, the EM frequency of the EM stimulation applied varies based on the tinnitus frequency F of the patient.

Preferably, the EM frequency comprises further frequencies in a frequency range around the tinnitus frequency F received. In one embodiment, the EM frequency comprises a larger frequency band around the received tinnitus frequency than the received tinnitus frequency. In another embodiment, the tinnitus frequency received already comprises the (non-zero) frequency band around the center frequency of the tinnitus frequency (or of each tinnitus tone). The frequency range/band corresponds to a frequency spectrum around the (center) tinnitus frequency/tone which corresponds to the change of tone which is not noticeable by the ear 4. The frequency range is preferably smaller than 1%, preferably 0,75%, preferably than 0,5% of the tinnitus frequency. If the tinnitus frequency comprises a plurality of tones, the EM frequency comprises a plurality of different frequencies (corresponding to noticeable different tones), each having a corresponding frequency range around the different frequencies. In a preferred embodiment, the frequency range is larger for higher frequency tones than for smaller frequency tones. This can be achieved by a size of the frequency range calculated based on the tinnitus frequency (e.g. by the percentages given above) or simply by applying a larger absolute frequency range, when the tinnitus frequency increases above a certain frequency threshold.

In one embodiment, the EM frequency contains in addition to the tinnitus frequency a modulation with at least one modulation frequency between 0.5 Hz and 20 Hz, i.e. with a non-audible frequency. In one embodiment, the modulation frequency is chosen from the range between 0.5Hz and 2Hz, preferably between 0.8 Hz and 1.5 Hz, preferably between 0.9 Hz and 1.2 Hz. Such a modulation frequency corresponds to the frequency of the heartbeat. In one embodiment, the heartbeat frequency is measured, and the EM frequency is determined as the tinnitus frequency F received modulated with the measured heartbeat frequency. In one embodiment, the modulation frequency is chosen from the range between 5Hz and 15Hz, preferably between 7 Hz and 13 Hz, preferably between 8 Hz and 12 Hz. Such a modulation frequency corresponds to the frequency of the alpha waves. The modulation of the tinnitus frequency with the alpha and/or heartbeat frequencies showed even better results than the EM stimulation with the pure tinnitus frequency F.

The processing means 22 can be realized by a processor in one device or can be realized by a plurality of processors in the same or different devices, e.g. in a client server system as explained below in one example.

The control means 2 comprises further an output means 23 for controlling the EM generator to generate an EM stimulation with the determined EM frequency. The output means 2 generates and/or outputs the analogue electric signal to the (conversion means of the) EM generator 1. The output means 2 is preferably an analogue audio output with a driving circuit to generate the analogue audio signal based on a digital audio signal/file/message received from the processing means 22. The output means 2 comprises preferably an analogue audio output connector, like a TRS plug (commonly also called Jack) at the TRS plug. This allows to use any standard computing device 24 with an audio output like a smartphone as control means and to connect the EM generator 1 to the audio output 23 of the computing device 24. It is clear that the output means 23 can also be realized differently, i.e. not using standard audio equipment for producing the analogue electric signal driving the EM generator 1.

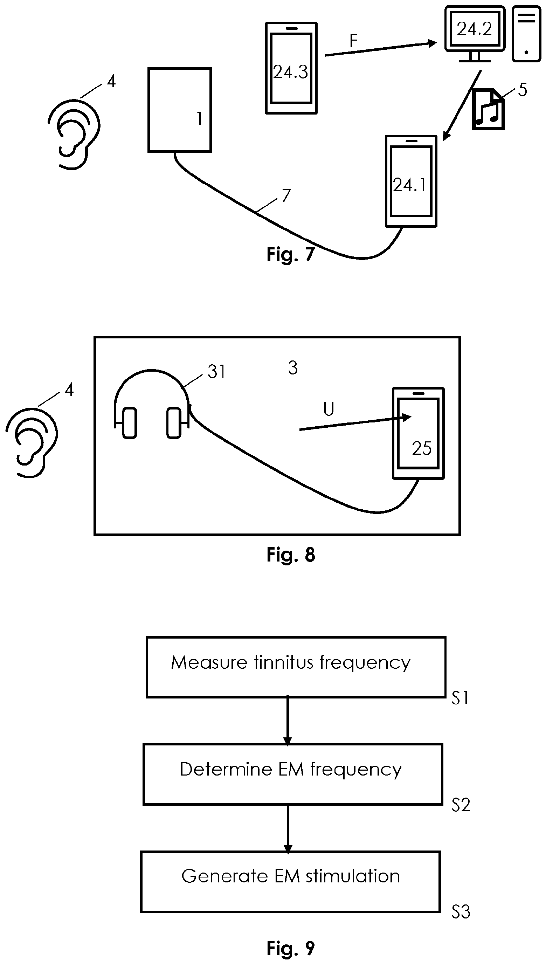

There are many ways two realize the system of the control means 2 and the EM generator 1. However, in a preferred embodiment, the system is realized by a computing device 24 and an EM generator 1 connected to the computing device 24. The functions of the control means 2 or the processing means 22 can be achieved by a software running on the computing device 24. Thus, the invention can be applied by the installation of a software on a computing device 24 and the connection of the EM generator 1 to the computing device 24. The computing device 24 can be realized as a standalone computing device 24 like a smartphone (as shown in Fig. 6), a tablet, a computer, etc. However, the computing device 24 can also be realized by a plurality of computing devices 24.1, 24.2 as shown in Fig. 7 performing together the functions of the control means 2 described above. The plurality of computing devices 24.1, 24.2 or the computing device 24 can comprise a client server system with a client device like a smartphone, tablet, computer, etc. to which the EM generator 1 is connected and a server 24.2 connected over a communication connection to the client device 24.1. The client device 24.1 is normally close to the patient/ear 4 and close to the EM generator 1, while the server 24.2 is normally remote from the client device 1. The communication connection is preferably an internet connection. The functions of the control means 2 can be distributed over the client device 24.1 and the server 24.2. The client device 24.1 would then realize at least the function of controlling the EM generator 1 to generate the EM stimulation, and, if the tinnitus frequency is obtained by a user input, also the function of the user input of the tinnitus frequency F. The server 24.2 has preferably a user management in which each patient is related to a user. Each user has user credentials stored in the server 24.2 to authenticate itself by the input of the user credentials. When the user authenticates itself by the user credentials in the client device 24.1, the actions of the client device 24.1 are related to the user account at the server 24.2. E.g. the tinnitus frequency F received at the client device 24.1 (and/or the EM frequency determined at the client device 24.1) would be sent to the server 24.2 and stored in the server 24.2 in relation to the user logged in the client device 24.1. The client device 24.1 could receive for the logged in user the stored EM frequency and/or tinnitus frequency so that the client device 24.1 could control a connected EM generator 1 to generate the EM stimulation at the EM frequency received (or generated based on the received tinnitus frequency). The last step is only necessary, if the client device 24.1 has not already stored the EM frequency or tinnitus frequency. The determination of the EM frequency based on the tinnitus frequency can be performed at the client device 24.1 or the server 24.2. The client device 24.1 can comprise also different client devices 24.1, 24.3 for different functions. E.g. a first client device 24.3 for inputting the tinnitus frequency F and a second client device 24.1 for controlling the EM stimulation. The server 24.2 is however not necessary and it is also possible to perform all functions at one local computing device 24. Once the EM frequency is determined in the control means 2 / computing device 24, preferably an audio signal is generated in the control means 2 / computing device 24. The audio signal can be generated at the moment of the treatment based on the information of the EM frequency or even based on the information of the tinnitus frequency F. The audio signal can also be generated well before and stored digitally in an audio file 5. The analogue audio signal would then simply be generated by replaying the audio file or the audio signal generated. The audio file 5 could be generated at the server 5 and transferred to the client device 24.1. The client device 24.1 could then use a standard audio software for replaying the audio file to apply the treatment and to give out the electric signal to the EM generator 1. Obviously, the client device 24.1 could also generate the audio signal based on the EM frequency at the moment of the treatment and give out the audio signal to the audio output of the client device 24.1.

In the examples of Fig. 6 and 7, the EM generator 1 is connected via a cable 7 to the audio output (as output means 23) of the computing device 24. If the computing device 24 is realized as a client server system, then the EM generator 1 is connected to the client device 24.1. The cable 7 is preferably a standard audio cable. The cable 7 is connected preferably to the analogue audio output of the computing device 24. The EM generator 1 is thus a peripheral device which can be connected over an analogue audio cable 7 to the analogue audio output 23 of the computing device 24. This embodiment is particularly advantageous as the EM generator 1 can be realized particularly simple as it requires only the EM conversion means and eventually some signal control components, e.g. a resistor to achieve the correct level of EM stimulation power in the EM conversion means. The remaining signal generation can use the standard audio equipment of the computing device 24.

Instead of connecting the EM generator 1 with a cable 7 transmitting an analogue signal, it is also possible to transmit the audio signal digitally to the EM generator 1. However, the EM generator 1 requires then a receiver for reading the digital audio data of the signal and a audio drive circuit to convert the digital data into the analogue audio signal. This would allow to transfer the audio signal via a digital signal in a digital cable connection or wirelessly, e.g. over Bluetooth (registered trademark). However, a cable connection is preferred to avoid other EM interferences of the ear 4 while performing the treatment. In this embodiment, the output means 23 is then realized in the peripheral device, because the analogue electric signal driving the EM conversion means is realized in the peripheral device.

The EM generator 1 (preferably realized in the peripheral device) can comprise a holding structure for holding the EM generator 1, in particular the EM conversion means, in a well-defined position with respect to the ear 4 so that the EM stimulation generated by the EM generator 1 generates a well-defined EM stimulation in the ear 4. However, it would also be possible to instruct the user/patient to hold the EM generator 1 in a certain distance from the ear 4 or to hold it at the ear during the treatment of the tinnitus. For the embodiments in Fig. 2, 4 and 5, the correct distance of the EM conversion means plays a role for the correct power of the electric field/signal induced in the nerve of the ear 4. For the embodiment in Fig. 3, the correct position of the electrode(s) on the ear 4 plays a role in the correct power of the electric signal applied to the nerve of the ear 4.

Even if the use of audio equipment for the generation of the analogue electric signal driving the EM generator 1 or EM conversion means is most preferred, it is also possible to use a dedicated signal circuitry for generating the electric signal driving the EM conversion means 1.

In a preferred embodiment, the EM generator 1, especially the EM conversion means is integrated in headphones, even more preferably, integrated in noise cancelling headphones. This allows the patient to see during the treatment with the EM stimulation, if the perceived sound of the tinnitus already becomes less or even disappears.

The treatment of the tinnitus works with different magnitudes of the EM stimulation. However, it is preferred to reduce the magnitude of the EM stimulation as far as possible without reducing the treatment effect. This is to avoid collateral damages of the EM stimulation in other parts of the patient. For an EM wave emitted by the antenna, the treatment shows a positive effect even with field strengths lower than 0.1 V/m (Volt per meter).

In one embodiment, the system comprises further a tinnitus measurement means 3 configured to measure the tinnitus frequency of the patient. This allows for providing the control means 2 with the tinnitus frequency F. The tinnitus measurement means is configured to output different audio tones in the audible frequency range and to receive a user input confirming that a certain tone corresponds to the tinnitus frequency. The frequency of the different audio tones output is controllable by a user input. For example, the user can select via a selector the audio tone output so that he can find finally the tinnitus frequency F of his ear 4. When the tone output corresponds finally to the tinnitus tone the user/patient perceives to hear in his ear 4, the tinnitus frequency F is found. In case of more complex tinnitus tones with a plurality of frequencies, the measurement means 3 is configured to output also such complex tones comprising a plurality of frequencies. The tinnitus measurement means 3 comprises in a preferred embodiment also a speaker, preferably headphones 31. If the EM generator 1 is integrated in headphones, the same headphones 31 can be used for the treatment with EM stimulation (using the output of EM stimulation by the EM generator 1) and for the measurement of the tinnitus frequency F (by using the output of sound by the headphones 31).

Preferably, the tinnitus measurement means 3 is realized as a measurement software in a computing device 25. The computing device 25 comprises a processor to run the measurement software and an output means for outputting the tones to speakers or headphones 31. Preferably, the same computing device 24 can be used as computing device 25. Preferably, the same common software is used for realizing the functions of the control means 2 and the function of the tinnitus measurement means 3. Preferably, the client software of the client device 24.1 combines the functions of the client device 24.1 described above and the tinnitus measurement means 3 or the computing device 25. In this case, the measurement means 3 and the control means 2 can be realized in the same client device 24.1 or computing device 24. In this case, the tinnitus frequency F measured by the measurement means 3 can automatically be input in the control means 2 (within the same software). However, it is also possible that the tinnitus measurement means 3 is realized in a distinct device/system, and the user reads out the measured tinnitus frequency F and provides this frequency F in the input means 21 of the control means 2 (manually).

Fig. 9 shows the steps of the method according to the invention.

In step S1, the tinnitus frequency F is measured. For example, as described above with the tinnitus measurement means 3.

In step S2, the EM frequency is determined based on the tinnitus frequency F measured. This can be realized for example as described for the control means 2, in particular as for the processing means 22.

In step S3, an EM stimulation at the determined EM frequency is generated with the EM generator 1. The generated EM stimulation is generated in the vicinity of the ear 4 or brain of the patient so that the nerves of the brain creating the tinnitus are treated with these EM stimulations.

It should be understood that the present invention is not limited to the described embodiments and that variations can be applied without going outside of the scope of the claims.

## Claims

1. System for treating a patient having tinnitus, comprising:
an EM generator (1) configured to generate an EM stimulation for the patient, wherein the EM stimulation is an electric and/or magnetic stimulation, wherein the EM generator (1) is configured to control an EM frequency of the EM stimulation,
a control means (2) configured
to receive a tinnitus frequency (F) of the patient,
to determine the EM frequency based on the received tinnitus frequency (F), and
to control the EM generator (1) to generate an EM stimulation based on the computed EM frequency.

2. System according to claim 1, wherein the EM frequency determined comprises the same frequency value in an EM frequency spectrum of the EM stimulation than the frequency value of the tinnitus frequency (F) in a sound frequency spectrum, wherein the same frequency value in the EM frequency spectrum is called EM tinnitus frequency (F).

3. System according to claim 2, wherein the EM frequency determined comprises a frequency range around the EM tinnitus frequency (F).

4. System according to claim 3, wherein the frequency range around the EM tinnitus frequency (F) range is larger, if the EM tinnitus frequency (F) is above a certain frequency, compared to a frequency range, when the EM tinnitus frequency (F) is below this certain frequency.

5. System according to any of claims 2 to 4, wherein the EM frequency determined comprises the EM tinnitus frequency (F) and at least one harmonic frequency of the EM tinnitus frequency (F).

6. System according to one of claims 1 to 5, wherein the EM generator (1) comprises an antenna (11) for generating an EM wave.

7. System according to one of claims 1 to 5, wherein the EM generator (1) comprises at least one electrode (12) to be brought in contact with the patient for applying an electric signal to the head of the patient.

8. System according to one of claims 1 to 5, wherein the EM generator (1) comprises a capacitive coupling means (13) for inducing an electric field in the head of the patient and/or an inductive coupling means for inducing a magnetic field in the in the head of the patient.

9. System according to one of claim 1 to 8, wherein the EM generator (1) is integrated in noise cancelling headphones.

10. System according one of claims 1 to 9, wherein the control means (2) is realized by a computing device (24) with an audio output, wherein the computing device (24) contains a software program configured, when executed on a processing means of the computing means, for outputting an audio signal with the determined EM frequency at the audio output, wherein the EM generator (1) is connected to the audio output to generate the EM stimulation based on the audio signal output from the audio output.

11. System according to claim 10, wherein the audio output is connected via a cable (7) to the EM generator (1), wherein the audio output is configured to output the audio signal as analogue audio signal, wherein EM generator (1) is driven by a signal based on the analogue audio signal.

12. System according to claim 10 or 11, wherein the computing device (24) comprises a smartphone with the audio output.

13. System according to one of claims 10 to 12, wherein the computing device (24) comprises a client server system with a server (24.2) and at least one client device (24.1, 24.3), wherein the at least one client device (24.1, 24.3) contains the audio output connected to the EM generator (1), wherein the server (24.2) comprises a user management for the management of different users authenticated by user credentials, wherein the at least one client device (24.1, 24.3) is associated with one of the users based on the user credentials received at the at least one client device (24.1, 24.3), wherein the tinnitus frequency (F) and/or the EM frequency determined is stored at the server (24.2) in relation to the user.

14. System according to one of the claims 1 to 13 comprising a tinnitus measurement means (3) configured to measure the tinnitus frequency (F) of the patient for providing the control means (2) with the tinnitus frequency (F).

15. System according to one of the claims 1 to 14, wherein the EM frequency comprises the EM tinnitus frequency (F) modulated with a frequency between 0,5 and 100Hz.
